# EUROPEAN PATENT APPLICATION

(11) **EP 4 563 693 A1**
(43) Date of publication of application: **04.06.2025**
(21) Application number: 23846402.8
(22) Date of filing: 21.07.2023
(51) Int. Cl.: C12N 1/00, C07K 14/415, C12N 5/02, C12N 5/10

(54) **ANIMAL CELL PROLIFERATION PROMOTER**

(30) Priority: 27.07.2022 JP 2022119238
(71) Applicant: Fuji Oil Holdings Inc., Izumisano-shi, Osaka 598-8540 (JP)
(72) Inventor: CHUNG, Hsuan, Tsukubamirai-shi, Ibaraki 300-2436 (JP); HIRANO, Keita, Izumisano-shi, Osaka 598-8540 (JP)
(74) Representative: Becker, Eberhard
(86) International application number: PCT/JP2023/026783
(87) International publication number: WO 2024/024671

(57) **Abstract**

The purpose of the present invention is to provide a material that can promote the proliferation of animal cells through addition to a serum-reduced culture medium. It was found that a plant protein material having a specified molecular weight distribution and NSI promoted the proliferation of animal cells through addition at a low concentration.

## Description

### Technical Field

The present invention relates to an animal cell proliferation promoter.

### Background Art

Efficiently proliferating cells in an animal cell culture broadens the scope of research and development in various fields. For example, research and development is anticipated to efficiently advance in the fields of regenerative medicine and cultured meat using cells. In addition, such cell proliferation can improve the productivity of substances necessary for sustaining life, such as hormones, antibodies, and enzymes.

As a known technique, the addition of a growth factor such as a serum derived from a cow, horse, or other animal or of a component derived from an animal serum to a culture medium is generally used as an operating standard.

In recent years, the field of regenerative medicine and the research field of cultured meat using cells have been attracting attention, but in these fields, concerns remain about problems such as the safety of unnecessary substances contained in animal serum and for which the composition is been fully clarified, as well as quality stability, which varies by individual animal.

Therefore, developments of serum-free culture media and chemically synthesized growth factors have advanced in recent years, and these growth factors are being used and added to culture media in place of animal serum.

However, components serving as growth factors used in culture media in place of animal serum are chemically synthesized and expensive. Other problems include the fact that these components are not edible, and that because the growth factor is expensive, production costs are high, and use in the development of cultured meat that could become a new option as a future food source is limited. In addition, although amino acids and low molecular weight peptides are added to the culture medium as nitrogen sources, these components contribute to cell proliferation as nutrients for cells, and it is difficult to substitute all the functions of serum using only low molecular weight components.

Patent Document 1 proposes a method for increasing the cell proliferation rate by adding a soybean protein hydrolysate and a yeast extract to a serum-free culture medium. Patent Document 2 discloses a technique relating to a culture medium for culturing animal cells, the culture medium containing a hydrolysate of a β-conglycinin concentrate.

### Citation List

### Patent Documents

Patent Document 1: JP 2002-520014 T
Patent Document 2: JP 2011-182736 A

### Summary of Invention

### Technical Problem

Thus, an object of the present invention is to provide a material that can promote the proliferation of animal cells when added to a culture medium in which the serum is reduced. Solution to Problem

The present inventors conducted intensive studies to solve the above problems. During an examination of various protein materials, the present inventors discovered that a protein material having a specific molecular weight distribution or nitrogen solubility index (NSI) and differing from a known soybean protein or the like promotes the proliferation of animal cells when added at a low concentration, and thereby the present inventors arrived at the present invention.

That is, the present invention provides the following aspects.
(1) An animal cell proliferation promoter containing a protein material having all of the following characteristics a) to c):
   a) the protein content in the solid content is 70 mass% or greater;
   b) the nitrogen solubility index (NSI) is 80 or greater; and
   c) in measurement results of the molecular weight distribution, an area ratio of the protein material having 2000 Da or greater and less than 20000 Da is 30% or greater, and an area ratio of the protein material having 20000 Da or greater is 70% or less.
(2) The animal cell proliferation promoter according (1), wherein, in measurement results of the molecular weight distribution, the area ratio of the protein material having 2000 Da or greater and less than 20000 Da is from 45% to 90%.
(3) The animal cell proliferation promoter according to (2), wherein, in measurement results of the molecular weight distribution, an area ratio of the protein material having less than 2000 Da is 45% or less.
(4) An animal cell culture medium containing, as a protein material, the animal cell proliferation promoter described in (1) at an amount of 0.015 wt.% or greater in the culture medium.
(5) An animal cell culture medium containing, as a protein material, the animal cell proliferation promoter described in (2) at an amount of 0.015 wt.% or greater in the culture medium.
(6) An animal cell culture medium containing, as a protein material, the animal cell proliferation promoter described in (3) at an amount of 0.015 wt.% or greater in the culture medium.
(7) The animal cell culture medium according to (4), wherein the animal cell culture medium is a serum-reduced culture medium in which an amount of serum in the culture medium is less than 10%.
(8) The animal cell culture medium according to (5), wherein the animal cell culture medium is a serum-reduced culture medium in which an amount of serum in the culture medium is less than 10%.
(9) The animal cell culture medium according to (6), wherein the animal cell culture medium is a serum-reduced culture medium in which an amount of serum in the culture medium is less than 10%.
(10) The animal cell culture medium according to (7), wherein the serum-reduced culture medium is a serum-free culture medium.
(11) The animal cell culture medium according to (8), wherein the serum-reduced culture medium is a serum-free culture medium.
(12) The animal cell culture medium according to (9), wherein the serum-reduced culture medium is a serum-free culture medium.
(13) A method for culturing animal cells, the method including culturing animal cells in the animal cell culture medium described in (4).
(14) A method for culturing animal cells, the method including culturing animal cells in the animal cell culture medium described in (5).
(15) A method for culturing animal cells, the method including culturing animal cells in the animal cell culture medium described in (6).
(16) A method for culturing animal cells, the method including culturing animal cells in the animal cell culture medium described in (7).
(17) A method for culturing animal cells, the method including culturing animal cells in the animal cell culture medium described in (10).
(18) A method for promoting the proliferation of animal cells, the method including adding the animal cell proliferation promoter described in (1) to an animal cell culture medium, and then culturing animal cells.
(19) A method for promoting the proliferation of animal cells, the method including adding the animal cell proliferation promoter described in (1) to a serum-reduced animal cell culture medium with the amount of serum in the culture medium being less than 10%, and then culturing animal cells.
(20) The method for promoting the proliferation of animal cells according to (19), wherein the serum-reduced animal cell culture medium is a serum-free animal cell culture medium. Advantageous Effects of Invention

According to the present invention, the proliferation of animal cells can be promoted in a serum-reduced culture medium.

### Brief Description of Drawings

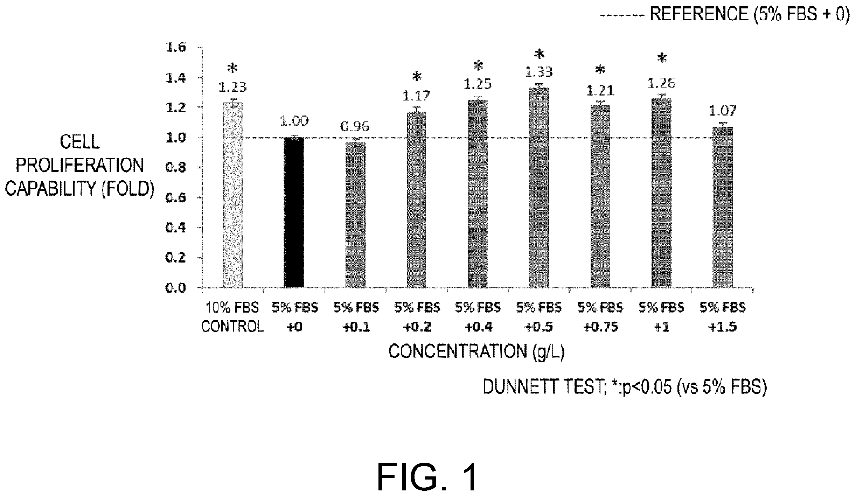

FIG. 1 is a graph showing the results of a cell proliferation capability test using a plant protein material A and the C2C12 myoblast cell line derived from the striated muscle of a mouse. The vertical axis of the graph represents the cell proliferation capability (fold) of test groups based on the proliferation capability of the C2C12 myoblast cell line derived from the striated muscle of a mouse being 1, the C2C12 myoblast cell line being cultured in Dulbecco's Modified Eagle Medium (DMEM) containing 5% fetal bovine serum (FBS) and used as a basal culture medium.

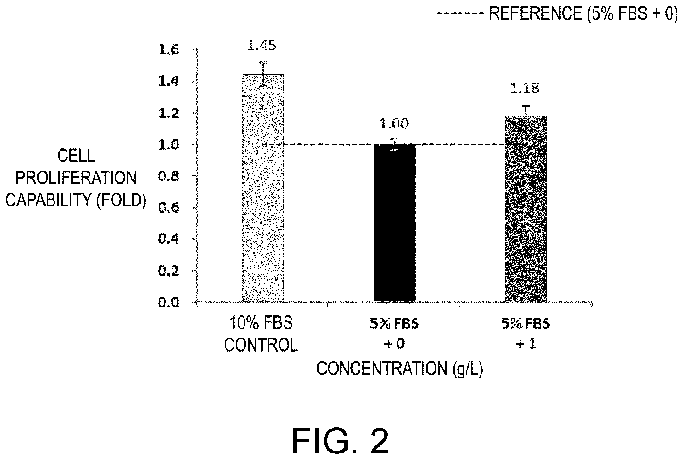

FIG. 2 is a graph showing the results of a cell proliferation capability test using a plant protein material B and the C2C12 myoblast cell line derived from the striated muscle of a mouse. The vertical axis of the graph represents the cell proliferation capability (fold) of test groups based on the proliferation capability of the C2C12 myoblast cell line derived from the striated muscle of a mouse being 1, the C2C12 myoblast cell line being cultured in Dulbecco's Modified Eagle Medium (DMEM) containing 5% fetal bovine serum (FBS) and used as a basal culture medium.

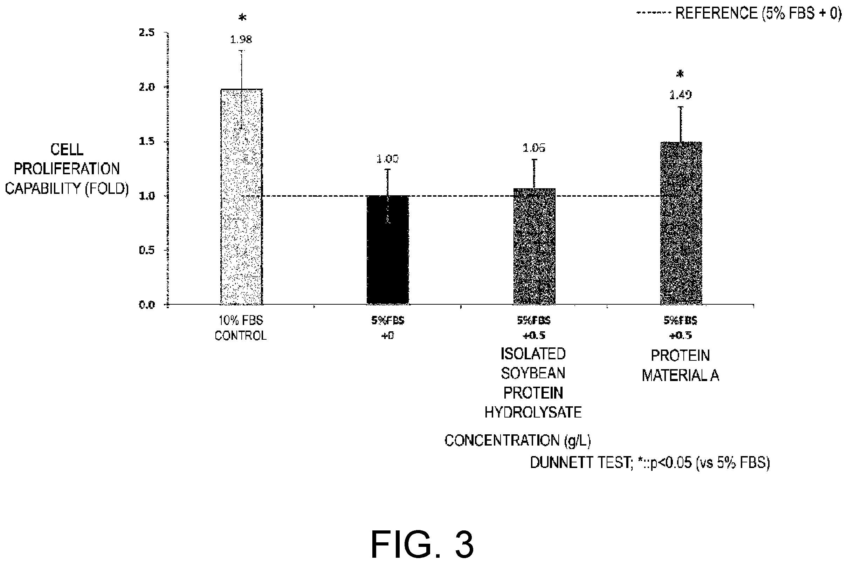

FIG. 3 is a graph showing the results of a cell proliferation capability test using the plant protein material A or an isolated soybean protein hydrolysate, and the C2C12 myoblast cell line derived from the striated muscle of a mouse. The vertical axis of the graph represents the cell proliferation capability (fold) of test groups based on the proliferation capability of the C2C12 myoblast cell line derived from the striated muscle of a mouse being 1, the C2C12 myoblast cell line being cultured in Dulbecco's Modified Eagle Medium (DMEM) containing 5% fetal bovine serum (FBS) and used as a basal culture medium.

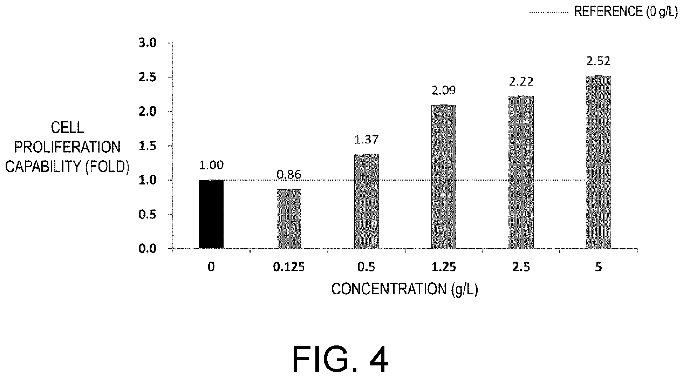

FIG. 4 is a graph showing the results of a cell proliferation capability test using the plant protein material A and the human proximal tubular cell line HK2. The vertical axis of the graph represents the cell proliferation capability (fold) of test groups based on the proliferation capability of the human proximal tubular cell line HK2 being 1, with the human proximal tubular cell line HK2 being cultured in a BIO-MPM-1 serum-free culture medium used as a basal culture medium. Description of Embodiments

### (Animal Cell Proliferation Promoter)

An animal cell proliferation promoter of the present invention is characterized by containing a plant protein material having all the following characteristics (a) to (c):
a) the content of protein in the solid content is 70 mass% or greater;
b) the nitrogen solubility index (NSI) is 80 or greater; and
c) in measurement results of the molecular weight distribution, an area ratio of the protein material having 2000 Da or greater and less than 20000 Da is 30% or greater, and an area ratio of the protein material having 20000 Da or greater is 70% or less.

In the solid content of the animal cell proliferation promoter of the present invention, the content of the plant protein material is preferably 50 mass% or greater, more preferably 60 mass% or greater, 70 mass% or greater, 80 mass% or greater, 90 mass% or greater, 95 mass% or greater, or 100 mass%.

### (Protein Material)

The protein material of the present invention may be a plant protein material or an animal protein material, and use of a plant protein material is preferable.

The concept of the protein material of the present invention is a food material that contains a plant protein or an animal protein as a main component and that is used as a raw material of various processed foods and beverages. Examples of the plant protein material include legumes such as soybeans, peas, mung beans, lupin beans, chickpeas, kidney beans, lentil beans, and cowpeas; seeds such as sesame seeds, canola seeds, coconut seeds, and almond seeds; grains such as corn, buckwheat, wheat, and rice; vegetables; and fruits. In a more specific embodiment, the plant protein material is prepared from a legume protein. In a still more specific embodiment, the plant protein material is prepared from soybean protein, pea protein, mung bean protein, or broad bean protein. In an even more specific embodiment, the plant protein material is prepared from soybean protein or pea protein. For example, a protein material derived from soybean is prepared by further concentrating and processing a protein from a soybean raw material such as defatted soybean or whole soybean, and in general, examples thereof conceptually include soybean protein isolates, soybean protein concentrates, soybean milk powder, and various processed products thereof.

Examples of the animal protein material include milk proteins such as casein, sodium caseinate, and whey protein, and egg whites.

A plant protein material and an animal protein material may be used in combination. When plant and animal proteins are used in combination, the mixing ratio is not particularly limited, but the ratio of the plant protein material to the animal protein material is from 99:1 to 1:99.

The protein material of the present invention will be further described using a plant protein material as an example.

### a) Protein Purity

In a plant protein material of the present aspect and used in an animal cell culture medium, the protein content in the solid content is 70 mass% or greater, such as, for example, 80 mass% or greater, 85 mass% or greater, or 90 mass% or greater. A raw material of the plant protein material included in the above range is preferably a protein isolate, and in a case of preparation from a protein material derived from soybeans, examples of the raw material include a soybean protein isolate.

### <Measurement of Protein Purity>

The protein purity is measured by the Kjeldahl method. Specifically, the mass of nitrogen measured by the Kjeldahl method relative to the mass of a protein material dried at 105°C for 12 hours is expressed by the "mass%" of the protein content in the dried product. The nitrogen conversion coefficient is 6.25. Basically, the protein purity is obtained by rounding the value to the first decimal place.

### b) NSI of Protein

The plant protein material used in the animal cell culture medium of the present aspect has a nitrogen solubility index (NSI), which is used as an index of protein solubility, of 80 or higher. More preferably, a plant protein material having an NSI of 85 or higher, 90 or higher, 95 or higher, or 97 or higher can be used. For example, as a plant protein material having a high NSI, a plant protein material that has not been subjected to a treatment for insolubilizing the protein, such as an enzymatic decomposition treatment or a mineral addition treatment, or a plant protein material that has been subjected to such a treatment only to a small extent is preferably used.

A high NSI of the plant protein material indicates high dispersibility in water and can contribute to dispersion stability of the animal cell culture medium of the present aspect. When the NSI of the plant protein material is too low, precipitation tends to occur, which is not preferred.

Note that the NSI is expressed as a proportion (mass%) of water-soluble nitrogen (crude protein) to a total nitrogen amount based on a method described below. Moreover, in the present invention, the NSI is a value measured in accordance with the below-described method.

### <NSI Measurement Method>

An amount of 60 mL of water is added to 3 g of a sample and stirred with a propeller at 37°C for 1 hour, after which the solution is centrifuged at 1400 × g for 10 minutes, and a supernatant (I) is collected. Next, 100 mL of water is again added to the remaining precipitate, and the mixture is again stirred with a propeller at 37°C for 1 hour and then centrifuged to collect a supernatant (II). The supernatants (I) and (II) are combined, and water is added to the mixed liquid to obtain a volume of 250 mL. The solution is then filtered with filter paper (No. 5), and then the nitrogen content (water-soluble nitrogen) in the filtrate is measured by the Kjeldahl method. At the same time, the total nitrogen amount in the sample is measured by the Kjeldahl method, and the percentage of the water-soluble nitrogen to the total nitrogen amount is expressed in mass% and is defined as the NSI. Basically, the value is obtained by rounding the value to the first decimal place.

### c) Molecular Weight Distribution

When the molecular weight is measured by gel filtration, the plant protein material used in the animal cell culture medium of the present aspect has an area ratio of the molecular weight distribution of 30% or greater for a range of 2000 Da or greater and less than 20000 Da and an area ratio of 70% or less for a range of 20000 Da or higher. In a specific embodiment, the area ratio is 35% or greater for a range of 2000 Da or greater and less than 20000 Da and is 65% or less for a range from 20000 Da or higher.

In a certain embodiment, when the molecular weight of the plant protein material is measured by gel filtration, the area ratio of the molecular weight distribution for a range of 2000 Da or greater and less than 10000 Da is from 10 to 40%, such as, for example, from 10 to 35%, from 15 to 35%, from 10 to 30%, or from 20 to 30%; and for a range of 10000 Da or greater, the area ratio is from 50 to 80%, such as, for example, from 55 to 75%, from 60 to 75%, from 60 to 70%, or from 65 to 75%. In a more specific embodiment, the area ratio of the protein material having less than 2000 Da is 15% or less, for example, 5% or less, 13% or less, 9% or less, 8% or less, or 7% or less, and the lower limit is, for example, 0% or greater, 1% or greater, 1.5% or greater, 2% or greater, or 3% or greater.

In another certain embodiment, in molecular weight distribution when the molecular weight is measured by gel filtration, the plant protein material has an area ratio for a range of 2000 Da or greater and less than 20000 Da of from 45 to 90%, such as, for example, from 50 to 85%, from 55 to 80%, from 55 to 75%, or from 60 to 70%.

In an even more specific embodiment, the area ratio of the protein material having less than 2000 Da is 45% or less, such as, for example, 40% or less, 35% or less, or 33% or less, and the lower limit is, for example, 0% or greater, 1% or greater, 2% or greater, 5% or greater, 10% or greater, or 15% or greater. In addition, in a still more specific embodiment, the area ratio of the protein material having 10000 Da or greater is less than 50%, such as, for example, from 5 to 45%, from 10 to 40%, or from 12 to 35%. In yet another specific embodiment, the area ratio of the protein material having 20000 Da or greater is less than 55%, such as, for example, 50% or less, 40% or less, 30% or less, 25% or less, 20% or less, or 15% or less.

The molecular weight distribution of the plant protein material in such ranges indicates that a moderately low molecular weight protein is a main component, while an amount of a highly degraded low molecular weight peptide is small. The molecular weight distribution is measured on the basis of a below-described method.

### <Molecular Weight Distribution>

The molecular weight distribution is measured through high-performance liquid chromatography (HPLC), that is, a protein material is adjusted to a concentration of 0.1 mass% using an eluent and then filtered through a 0.2 µm filter, and the obtained product is used as a sample solution. A gel filtration system is assembled by connecting two kinds of columns in series, a known protein or the like serving as a molecular weight marker and described in Table 1 is first charged, and a calibration curve is created from the relationship between the molecular weight and the retention time. The sample solution is then charged, and the content percentage of each molecular weight fraction is determined by the proportion of the area of a specific molecular weight range (time range) to the area of the absorbance chart for the entire chromatograph (1st column: "TSK gel G3000SW_{XL}" (Sigma-Aldrich), 2nd column: "TSK gel G2000SW_{XL}" (Sigma-Aldrich); eluent: a mixture of 1% SDS, 1.17% NaCl, and a 50 mM phosphate buffer (pH 7.0); 23°C; flow rate: 0.4 mL/min; detection: UV 220 nm).

**(Table 1)**

| Molecular Weight Marker | |
|---|---|
| Marker | Molecular Weight |
| Thyroglobulin | 335000 |
| γ-globulin | 150000 |
| Albumin | 67000 |
| Peroxidase | 43000 |
| Myoglobin | 18000 |
| Cytochrome C | 12384 |
| Insulin | 5734 |
| Glutathione | 307 |
| p-amino acid benzoic acid | 137 |

### ("Degradation and Molecular Weight Distribution Adjustment Treatment" of Plant Protein Material)

The plant protein material used in the animal cell culture medium of the present aspect can be obtained by combining a "degradation treatment" for degrading a protein and a "molecular weight distribution adjustment treatment" for adjusting the molecular weight distribution of the protein and using the combined "degradation and molecular weight distribution adjustment treatment". Examples of the "degradation treatment" include an enzyme treatment, a pH adjustment treatment (e.g., an acid treatment or an alkali treatment), a heating treatment, a cooling treatment, a high-pressure treatment, an organic solvent treatment, a mineral addition treatment, a supercritical treatment, an ultrasonic treatment, an electrolysis treatment, and combinations thereof. Examples of the "molecular weight distribution adjustment treatment" include filtration, gel filtration, chromatography, centrifugation, electrophoresis, dialysis, and combinations thereof. The order and number of times of the "degradation treatment" and the "molecular weight distribution adjustment treatment" are not particularly limited; the "degradation treatment" may be carried out before the "molecular weight distribution adjustment treatment", the "molecular weight distribution adjustment treatment" may be carried out before the "degradation treatment", or both treatments may be carried out at the same time. In addition, for example, the "degradation treatment" may be carried out between two or more times of the "molecular weight distribution adjustment treatment", the "molecular weight distribution adjustment treatment" may be carried out between two or more times of the "degradation treatment", or each treatment may be carried out a plurality of times in any order. In a case in which a desired molecular weight distribution is obtained by the "degradation treatment", the "molecular weight distribution adjustment treatment" need not be carried out. When these treatments are combined and carried out a plurality of times, all the treatments may be carried out continuously from the raw material stage or may be carried with intervals therebetween. For example, a commercially available product that has undergone a certain treatment may be used as a raw material and subjected to another treatment. As long as the above characteristics are satisfied, the specific plant protein material may be obtained by mixing a plant protein material that has undergone the molecular weight distribution adjustment treatment with a protein that has not undergone the molecular weight distribution adjustment treatment. In this case, a ratio of the two protein materials (the protein material that has undergone the treatment : the protein that has not undergone the treatment) can be adjusted as appropriate within a range that satisfies the characteristics described above, and in terms of a mass ratio, the ratio thereof may be, for example, from 1:99 to 99:1, such as, for example, from 50:50 to 95:5, or from 75:25 to 90:10. In a certain embodiment, the plant protein material used in the animal cell culture medium of the present aspect is composed of a plant protein material that has undergone the "degradation and molecular weight distribution adjustment treatment".

In the treatment for degrading or denaturing a protein, conditions such as, for example, the enzyme, the pH, the types and concentrations of an organic solvent and a mineral, the temperature, the pressure, the output intensity, the current, and the time, can be appropriately set by a person skilled in the art. In a case in which an enzyme is used, examples of the enzyme to be used include proteases that are classified into "metal proteases", "acidic proteases", "thiol proteases", and "serine proteases". The reaction can be carried out at a reaction temperature of from 20 to 80°C and preferably of from 40 to 60°C. In the case of a pH adjustment treatment, the treatment can be carried out in a pH range in which any pH values of 2, 2.5, 3, 3.5, 4, 4.5, 5, 5.5, 6, 6.5, 7, 7.5, 8, 8.5, 9, 9.5, 10, 10.5, 11, 11.5, and 12 are set as the upper limit and the lower limit, and for example, the pH range may be from 2 to 12. In the case of an acid treatment, the method may be a method of adding an acid or a method of carrying out fermentation treatment, such as lactic acid fermentation. Examples of the acid to be added include inorganic acids, such as hydrochloric acid and phosphoric acid; and organic acids, such as acetic acid, lactic acid, citric acid, gluconic acid, phytic acid, sorbic acid, adipic acid, succinic acid, tartaric acid, fumaric acid, malic acid, and ascorbic acid. Alternatively, an acid may be added using a food or drink containing an acid, such as a fruit juice of lemon or the like, a concentrated fruit juice, fermented milk, yogurt, or a brewed vinegar. In the case of an alkali treatment, an alkali, such as sodium hydroxide or potassium hydroxide, can be added. In the case of a denaturant treatment, a denaturant, such as guanidine hydrochloride, urea, arginine, or PEG, may be added. In the case of a heating or cooling treatment, examples of the heating temperature include temperature ranges that include any temperatures of 60°C, 70°C, 80°C, 90°C, 100°C, 110°C, 120°C, 125°C, 130°C, 135°C, 140°C, 145°C, and 150°C set as the upper limit and the lower limit, and for example, the temperature range may be from 60°C to 150°C. Examples of the cooling temperature include temperature ranges including any temperatures of -10°C, -15°C, -20°C, -25°C, -30°C, -35°C, -40°C, -45°C, -50°C, -55°C, -60°C, - 65°C, -70°C, and -75°C set as the upper limit and the lower limit, and for example, the temperature range may be from -10°C to -75°C. Examples of the heating or cooling time include time ranges including any times of 5 seconds, 10 seconds, 30 seconds, 1 minute, 5 minutes, 10 minutes, 20 minutes, 30 minutes, 40 minutes, 50 minutes, 60 minutes, 70 minutes, 80 minutes, 90 minutes, 100 minutes, 120 minutes, 150 minutes, 180 minutes, and 200 minutes set as the upper limit and the lower limit, and for example, the time range may be from 5 seconds to 200 minutes. In the case of a high-pressure treatment, examples of the pressure condition include pressure ranges including any pressures of 100 MPa, 200 MPa, 300 MPa, 400 MPa, 500 MPa, 600 MPa, 700 MPa, 800 MPa, 900 MPa, and 1000 MPa set as the upper limit and the lower limit, and for example, the pressure range may be from 100 MPa to 1000 MPa. In the case of an organic solvent treatment, examples of the solvent to be used include alcohols and ketones, such as, for example, ethanol and acetone. In the case of a mineral addition treatment, examples of the mineral to be used include divalent metal ions, such as calcium and magnesium. In the case of a supercritical treatment, the treatment can be implemented, for example, using carbon dioxide in a supercritical state at a temperature of about 30°C or higher and a pressure of about 7 MPa or higher. In the case of an ultrasonic treatment, the treatment can be carried out, for example, by irradiation at a frequency of from 100 KHz to 2 MHz with an output of from 100 to 1000 W. In the case of an electrolysis treatment, for example, an aqueous protein solution may be treated by application of a voltage of from 100 mV to 1000 mV. In a specific embodiment, a treatment for degrading a protein is selected from a heating treatment and combinations thereof.

In the treatment for adjusting the molecular weight distribution of a protein, conditions such as, for example, the type of filter medium, the carrier for gel filtration, the centrifugal rotation speed, the electric current, and the time, can be appropriately set by a person skilled in the art. Examples of the filter medium include filter paper, filter cloth, diatomaceous earth, ceramics, glass, and membranes. Examples of the carrier for gel filtration include dextran and agarose. Examples of the conditions of centrifugation include conditions of from 1000 to 3000 × g and of from 5 to 20 minutes.

Note that commercially available soybean protein materials known in the art, such as "Fujipro E", "Fujipro CL", "Fujipro AL", "New Fujipro 4500", "Proleena RD-1", "Proleena 900", and "Proleena HD101R", do not correspond to a plant protein material satisfying all the above characteristics of a) to c).

### (Animal Cell Culture Medium)

The animal cell culture medium of the present invention contains the abovementioned animal cell proliferation promoter.

The animal cell culture medium of the present invention is preferably a culture medium in which serum has been reduced.

One embodiment of the culture medium with reduced serum is a serum-reduced culture medium. In the present invention, a serum-reduced culture medium is a culture medium in which the serum is reduced as compared with a normal serum culture medium, and the amount of serum in the culture medium is preferably less than 10%, and more preferably 9.5%, 9%, 8%, 7%, 6%, 5%, 4%, 3%, 2%, 1%, 0.5%, 0.2%, 0.1% or the like.

Another embodiment of the serum-reduced culture medium is a serum-free culture medium.

The type of the culture medium is not particularly limited, and a commercially available culture medium can be used. A preferable culture medium varies depending on the type of animal cells to be cultured, and can be appropriately selected by a person skilled in the art.

Examples of animal cells include fibroblasts, epidermal cells, mammary gland cells, adipocytes, myoblasts, smooth muscle cells, endothelial cells, osteoblasts, hepatocytes, vascular endothelial cells, and precursor cells thereof. Other examples include somatic stem cells such as hematopoietic stem cells, mesenchymal stem cells, and neural stem cells, as well as pluripotent stem cells, and induced pluripotent stem cells.

Other examples of animal cells include those that produce useful substances such as antibodies, enzymes (urokinase, etc.), hormones (insulin, etc.), cytokines (interferons, interleukins, tumor necrosis factors, colony stimulating factors, growth factors, etc.), and other physiologically active proteins and peptides, and examples of such animal cells include nontransformed cells such as skin cells, cartilage cells, hepatocytes, pancreatic cells, and renal cells, and transformed cells into which coding genes or genes involved in the biosynthesis of useful substances have been introduced. Examples of the transformed cells include antibody-producing cells such as mouse myeloma cells and Chinese hamster ovary cells. These useful substances can be efficiently produced by culturing these animal cells. In addition, these useful substances can also be used in the field of regenerative medicine by culturing and efficiently proliferating skin cells, cartilage cells, hepatocytes, pancreatic cells, ES cells, iPS cells and the like.

Animal cells are preferably derived from mammals, such as mice, rats, rabbits, humans, horses, cows, monkeys, pigs, chickens, ducks, dogs, sheep, cats, and goats.

The amount of the animal cell proliferation promoter of the present invention in the culture medium varies depending on the type of animal cells to be cultured, but is desirably 0.015 mass% or greater in terms of the protein material in the animal cell culture medium. The amount thereof is preferably 0.02 mass% or greater, or 0.04 mass% or greater. The upper limit is preferably 0.5 mass% or less, more preferably 0.4 mass% or less, and even more preferably 0.3 mass% or less, and can be set to 0.2 mass% or less, 0.15 mass% or less, or 0.1 mass% or less. Specific examples of the range of the amount of the animal cell proliferation promoter in the culture medium include from 0.015 to 0.5 mass%, from 0.015 to 0.4 mass%, from 0.015 to 0.3 mass%, from 0.015 to 0.2 mass%, from 0.015 to 0.15 mass%, from 0.015 to 0.1 mass%, from 0.02 to 0.5 mass%, from 0.02 to 0.4 mass%, from 0.02 to 0.3 mass%, from 0.02 to 0.2 mass%, from 0.02 to 0.15 mass%, from 0.02 to 0.1 mass%, from 0.04 to 0.5 mass%, from 0.04 to 0.4 mass%, from 0.04 to 0.3 mass%, from 0.04 to 0.2 mass%, from 0.04 to 0.15 mass%, and from 0.04 to 0.1 mass%. When the protein material of the present invention is contained at an amount within the above range, proliferation of the animal cell culture can be promoted.

In particular, as described above, the protein material of the present invention is excellent in that the proliferation of animal cells can be promoted even at a low concentration of 0.1 mass% or less.

In the present invention, promoting the proliferation of animal cells means that the proliferation of cells cultured in a culture medium supplemented with the protein material of the present invention is higher than the proliferation of cells cultured in a culture medium not supplemented with the protein material.

More specifically, promoting the proliferation of animal cells means that the cell proliferation capability of animal cells cultured in a culture medium supplemented with a plant protein material is 1.07 or greater based on a cell proliferation capability of 1 of animal cells cultured in a culture medium not supplemented with the plant protein material. The cell proliferation capability of animal cells cultured in a culture medium supplemented with a plant protein material is preferably 1.08 or greater, more preferably 1.10 or greater, even more preferably 1.13 or greater, and still more preferably 1.15 or greater.

### A method for measuring cell proliferation will be described later.

### (Effect of Promoting Animal Cell Proliferation)

When animal cells are cultured in the animal cell culture medium of the present invention, cell proliferation of the animal cells is promoted by the protein material of the present invention. Thus, if the animal cell whose proliferation is promoted can produce a substance useful for the production of cultured meat, production of cultured meat by the animal cell can also be promoted.

Moreover, when the animal cell whose proliferation is promoted is a type of animal cell that produces a substance useful for the human body, production of the useful substance by the animal cell can also be promoted. Examples of various useful substances that can be produced from animal cells include antibodies, enzymes, and hormones.

### Examples

Hereinafter, the present invention is described by way of examples. Note that, in the examples, all parts and percentages are based on mass unless otherwise specified.

### (Preparation of Plant Protein Materials)

The following plant protein materials were procured or prepared. The protein contents of these plant protein materials were all 80 mass% or greater. Detailed analytical values are presented in Table 2.

Plant protein material A: a product obtained by subjecting a soybean protein isolate to a degradation and molecular weight distribution adjustment treatment (a test product available from Fuji Oil Co., Ltd.; raw material soybean protein isolate: Fujipro F (a commercially available product from Fuji Oil Co., Ltd.))

Plant protein material B: a product obtained by subjecting a soybean protein isolate to a degradation and molecular weight distribution adjustment treatment (a test product available from Fuji Oil Co., Ltd.; raw material soybean protein isolate: Fujipro F (a commercially available product from Fuji Oil Co., Ltd.))

Isolated soybean protein hydrolysate: a commercially available product from Fuji Oil Co., Ltd.

**(Table 2)**

| | 20000 Da or greater | 2000 Da or greater and less than 20000 Da | Less than 2000 Da | 10000 Da or greater | NSI |
|---|---|---|---|---|---|
| Plant protein material A | 1.3 | 67.0 | 31.7 | 13.0 | 94.9 |
| Plant protein material B | 8.4 | 66.7 | 24.3 | 18.0 | 97.3 |
| Isolated soybean protein hydrolysate | n.d | 19.7 | 80.3 | 1.2 | 100 |

### (Example 1) Effect of Promoting Proliferation of Mouse Striated Muscle-Derived Myoblast Cells

The effect of the plant protein material A on promoting the proliferation of myoblast cells derived from mouse striated muscle was confirmed by the following method.

### (Cell Culturing Method)

Dulbecco's modified Eagle medium (hereinafter, referred to as DMEM) containing 5% fetal bovine serum (FBS) to which a Penicillin-Streptomycin-Amphotericin B Suspension was added to obtain a 100-fold dilution was prepared. Using this culture medium, a cell suspension was obtained in which the C2C12 myoblast cell line (hereinafter, referred to as C2C12) derived from the striated muscle of a mouse was adjusted to 5 × 10⁴ cells/mL. The obtained cell suspension was added to each of eight wells of a 96 well-plate at an amount of 0.1 mL/well. After equilibration at 37°C for 24 hours (CO₂ concentration = 5%), the culture medium was replaced with DMEM containing 5% FBS to which the plant protein material A of each concentration was added. This was then cultured at 37°C for 48 to 72 hours (CO₂ concentration = 5%), after which cell proliferation was measured.

Culturing was also carried out with DMEM containing 5% FBS or DMEM containing 10% FBS, to which no plant protein material was added.

To the DMEM containing 5% FBS, the plant protein material A was added at an amount of from 0.2 g/L (equivalent to 0.02%) to 1.5 g/L (equivalent to 0.15%).

### (Method for Evaluating Cell Proliferation Capability)

Cell proliferation was measured using the Cell Counting Kit-8 available from Dojindo Laboratories.

That is, water-soluble tetrazolium salt (WST-8) was added to each culture medium after culturing, and the mixture was incubated at 37°C for 60 minutes, after which the cell proliferation was measured by absorbance at 450 nm.

As the measurement principle, NADH produced by a dehydratase in the cells reduces the water-soluble tetrazolium salt (WST-8) to an orange-colored water-soluble formazan through an electron carrier. The absorbance at 450 nm of the formazan is proportional to the number of living cells, and thus the number of living cells can be measured.

The results are presented in FIG. 1.

As compared with the cell proliferation capability in the normal DMEM containing 10% FBS, the proliferation capability in the DMEM in which the concentration of FBS was reduced by half, that is, the DMEM containing 5% FBS, clearly decreased. On the other hand, when an amount from 0.2 g/L (equivalent to 0.02%) to 1.5 g/L (equivalent to 0.15%) of the plant protein material A was added to the DMEM containing 5% FBS, a significant recovery in the decreased proliferation capability occurred.

Therefore, it was confirmed that the plant protein material A has a proliferation effect on C2C12 in a serum-reduced culture medium.

### (Example 2)

Cells were cultured and the proliferation capability was evaluated in the same manner as in Example 1 with the exception that the plant protein material B was used as the plant protein material, and the addition amount to the DMEM containing 5% FBS was changed to 1.0 g/L. The results are presented in FIG. 2.

The plant protein material B was also confirmed to have a proliferation effect on C2C12 in a serum-reduced culture medium.

### (Example 3 and Comparative Example 1)

Cells were cultured and the proliferation capability was evaluated in the same manner as in Example 1 with the exception that the plant protein material A or isolated soybean protein hydrolysate was used as the plant protein material, and the addition amount to the DMEM containing 5% FBS was changed to 0.5 g/L. The results are presented in FIG. 3.

It was confirmed that the plant protein material A had a high proliferation effect on C2C12 in the serum-reduced medium (Example 3, indicated as protein material A in the figure), whereas the isolated soybean protein hydrolysate having a molecular weight distribution not satisfying the numerical values defined in the present invention exhibited a reduced proliferation effect on C2C12 (Comparative Example 1, indicated as the isolated soybean protein hydrolysate in the figure) and did not satisfy the acceptance criteria of the present invention.

### (Example 4 and Comparative Example 2)

The effect of the plant protein material of the present invention in a serum-free culture medium was confirmed by the following procedure.

A serum-free culture medium (BIO-MPM-1, available from Biological Industries Ltd.) containing 5 µM/mL of fibronectin (adhesive factor) to which glutamine was added to obtain a molarity of 2 mM was prepared. Using this serum-free culture medium, a cell suspension prepared by adjusting the human proximal tubular cell line HK2 to 1 × 10⁴ cells/mL was obtained. The obtained cell suspension was added to each of eight wells of a 96 well-plate at an amount of 0.1 mL/well. After equilibration at 37°C for 24 hours (CO₂ concentration = 5%), the medium was replaced with a serum-free culture medium (BIO-MPM-1) supplemented with the plant protein material A at each concentration. This was then cultured at 37°C for 48 to 72 hours (CO₂ concentration = 5%), after which cell proliferation was measured.

The plant protein material A was added to the BIO-MPM-1 serum-free culture medium at an amount of from 0.125 g/L (equivalent to 0.0125%) to 5 g/L (equivalent to 0.5%). In addition, cell proliferation capability was evaluated in the same manner as in Example 1. The evaluation results are presented in FIG. 4.

When the addition amount of the plant protein material A was 0.5 g/L, 1.25 g/L, 2.5 g/L, and 5 g/L (Example 4), it was confirmed that the proliferation effect on the human proximal tubular cell line HK2 was high in the serum-free culture medium.

## Claims

1. An animal cell proliferation promoter comprising a protein material having all of the following characteristics a) to c):
a) protein content in a solid content is 70 mass% or greater;
b) the nitrogen solubility index (NSI) is 80 or greater; and
c) in measurement results of the molecular weight distribution, an area ratio of the protein material having 2000 Da or greater and less than 20000 Da is 30% or greater, and an area ratio of the protein material having 20000 Da or greater is 70% or less.

2. The animal cell proliferation promoter according to claim 1, wherein, in measurement results of the molecular weight distribution, the area ratio of the protein material having 2000 Da or greater and less than 20000 Da is from 45% to 90%.

3. The animal cell proliferation promoter according to claim 2, wherein, in measurement results of the molecular weight distribution, an area ratio of the protein material having less than 2000 Da is 45% or less.

4. An animal cell culture medium comprising, as a protein material, the animal cell proliferation promoter described in claim 1 at an amount of 0.015 wt.% or greater in the culture medium.

5. An animal cell culture medium comprising, as a protein material, the animal cell proliferation promoter described in claim 2 at an amount of 0.015 wt.% or greater in the culture medium.

6. An animal cell culture medium comprising, as a protein material, the animal cell proliferation promoter described in claim 3 at an amount of 0.015 wt.% or greater in the culture medium.

7. The animal cell culture medium according to claim 4, wherein the animal cell culture medium is a serum-reduced culture medium in which an amount of serum in the culture medium is less than 10%.

8. The animal cell culture medium according to claim 5, wherein the animal cell culture medium is a serum-reduced culture medium in which an amount of serum in the culture medium is less than 10%.

9. The animal cell culture medium according to claim 6, wherein the animal cell culture medium is a serum-reduced culture medium in which an amount of serum in the culture medium is less than 10%.

10. The animal cell culture medium according to claim 7, wherein the serum-reduced culture medium is a serum-free culture medium.

11. The animal cell culture medium according to claim 8, wherein the serum-reduced culture medium is a serum-free culture medium.

12. The animal cell culture medium according to claim 9, wherein the serum-reduced culture medium is a serum-free culture medium.

13. A method for culturing animal cells, the method comprising culturing animal cells in the animal cell culture medium described in claim 4.

14. A method for culturing animal cells, the method comprising culturing animal cells in the animal cell culture medium described in claim 5.

15. A method for culturing animal cells, the method comprising culturing animal cells in the animal cell culture medium described in claim 6.

16. A method for culturing animal cells, the method comprising culturing animal cells in the animal cell culture medium described in claim 7.

17. A method for culturing animal cells, the method comprising culturing animal cells in the animal cell culture medium described in claim 10.

18. A method for promoting the proliferation of animal cells, the method comprising adding the animal cell proliferation promoter described in claim 1 to an animal cell culture medium, and then culturing animal cells.

19. A method for promoting the proliferation of animal cells, the method comprising adding the animal cell proliferation promoter described in claim 1 to a serum-reduced animal cell culture medium with the amount of serum in the culture medium being less than 10%, and then culturing animal cells.

20. The method for promoting the proliferation of animal cells according to claim 19, wherein the serum-reduced animal cell culture medium is a serum-free culture medium for culturing animal cells.
